Europäisches Patentamt

European Patent Office

Office européen des brevets

(11) Publication number:

**0 287 186**
**A2**

## EUROPEAN PATENT APPLICATION

(21) Application number: 88200737.0

(51) Int. Cl.⁴: **A61M 1/08**

(22) Date of filing: **18.04.88**

The title of the invention has been amended (Guidelines for Examination in the EPO, A-III, 7.3).

(30) Priority: **16.04.87 NL 8700909**

(43) Date of publication of application:
**19.10.88 Bulletin 88/42**

(84) Designated Contracting States:
**AT BE CH DE ES FR GB GR IT LI LU NL SE**

(71) Applicant: **Van der Vliet, Timotheus Theodorus**
**Westelijk Halfrond**
**NL-1183 HZ Amstelveen(NL)**

(72) Inventor: **Van der Vliet, Timotheus Theodorus**
**Westelijk Halfrond**
**NL-1183 HZ Amstelveen(NL)**

(74) Representative: **Baarslag, Aldert D. et al**
**Nederlandsch Octrooibureau Johan de**
**Wittlaan 15 P.O. Box 29720**
**NL-2502 LS Den Haag(NL)**

(54) **Device for incising and draining an abcess by suction.**

(57) Device for incising and draining an abcess by placing a holder around this abcess. By sucking up the skin locally it comes into contact with cutting means as a result of which the abcess is opened. By further applying vacuum the abcess is subsequently drained.

FIG 1

## Device for making an incision in suctionning of an abcess.

The present invention relates to a device for incising and draining an abcess.

The present method for removal of abcesses has the following disadvantages: in the first place, the treatment is relatively time-consuming, in the second place, there is a risk of infection for the treating physician, patient and any assistants since the infectious pus frequently gushes out in an uncontrolled manner under pressure during the incision, in the third place, it is often necessary to press gently on the skin surrounding the abcess in order to discharge the pus with the danger of subcutaneous spreading of the infection increasing, in the fourth place, all the instruments used have to be sterilised after the procedure and in the fifth place, it is possible for an incision to be made too large or larger than appears necessary as a result of which the patient is confronted with an unnecessarily large scar.

It is the object of the invention to avoid these disadvantages. This object is realised in an above-described device in that this comprises a holder which is at least partially open at one end and which is to be placed around the abcess, this holder being provided at the open end with cutting means which in the fitted state are at a distance from the abcess, and sucking means being present for providing a vacuum in this holder. By providing a vacuum after fitting the device on the abcess the skin is locally raised until it comes into contact with the cutting means and is subsequently cut open. As a result of the prevailing vacuum or by further applying vacuum the matter present in the abcess is then drained.

According to an advantageous embodiment of the device, the means which provide vacuum are a plunger/cylinder system. At the same time, the plunger/cylinder system preferably forms part of the holder. In order to avoid having to sterilise the device after each treatment the plunger is provided with a diaphragm which, together with the cylinder, is fitted in a simply detachable manner on the device. After each treatment the cylinder, together with the diaphragm, can be thrown away and a new clean assembly can be applied. Instead of or in addition to the diaphragm the plunger can be designed so as to be disposable. The plunger is then to be fitted on the device in a simply detachable manner.

According to a further advantageous embodiment the means for providing vacuum are equipped such that a first vacuum is applied during use of the device during a first phase and a second more considerable vacuum is applied during a second subsequent phase. The first vacuum is sufficient for providing an incision in the abcess while the second more considerable vacuum is necessary for draining the abcess completely.

According to a further advantageous embodiment the first vacuum is generated by spring means acting upon the plunger/cylinder system and the second vacuum is generated by lever means. A fixed given vacuum is provided by the spring means while a variable and more considerable vacuum is provided by the lever means as a result of the force applied thereon, which can be matched to suit the circumstances.

According to a further advantageous embodiment the device consists of sterilisable material. This applies, of course, not to the quickly detachable holder and the associated diaphragm if these are present.

According to a further embodiment of the device this comprises a pump housing and, fitted therein, a plunger rod provided on one end with a plunger and on the other end provided with a compression knob, the pump house being provided on the end next to the plunger with means for fixing a cylinder, spring means engaging on the plunger rod being present in the pump housing and the plunger rod being provided with a toothing to be engaged with a transport mechanism. At the same time locking means may be present for locking, under compression by the spring means, the plunger rod. The transport mechanism preferably comprises a lever mechanism engaging the toothing in one direction and means which hinder movement in the opposite direction.

The invention will be illustrated in detail hereinafter with reference to the exemplary embodiment shown in the drawing, in which

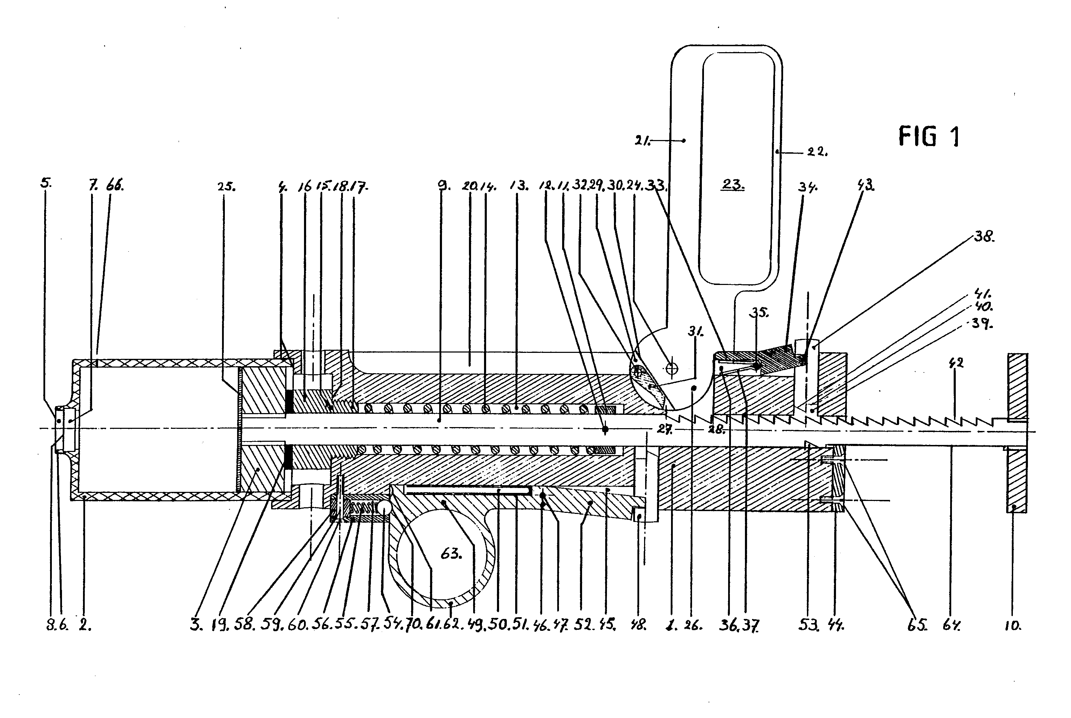

Figure 1 shows a longitudinal section of the instrument at the end of the treatment.

Figure 2 shows a longitudinal section of the instrument in its compressed state ready for operation at the start of the treatment.

The instrument according to the invention consists of a pump housing 1 made of durable material and having a cylindrical shape on one end of which a cylinder 2 made of transparent plastic is fitted.

This cylinder has an external diameter equal to the diameter of the cylindrical recess 4 in the pump housing and an internal diameter approximately equal to the external diameter of the plunger 3, as a result of which the cylinder can be pushed over the plunger until this cylinder is firmly clamped in the appropriate cylindrical recess 4. An easily exchangeable diaphragm 25 is fitted on the plunger 3. The cylinder is provided on its other end with a bottom having a round opening 5 therein and an up right edge 6 directed outwards. A small knife 7 is mounted diametrically in this opening with the cutting edge 8 directed outwards. The plunger 3 is fixed to the plunger rod 9. The plunger rod sticks in an axial bore in the pump housing and can move therein in the longitudinal direction of the pump housing. A compression knob 10 is mounted on the other end of the plunger rod. A cylindrical ring 11 which has an internal diameter equal to the diameter of the plunger rod and which is secured by a pin 12 by means of a clamp fit is mounted on the plunger rod 9. A bore 13 having a diameter larger than the external diameter of the cylindrical ring 11 and a length indicated in the drawing is provided in the pump housing, centrally and parallel to the plunger rod, which is partially provided with a screw thread at the cylinder side. In the annular space 13 which is formed between pump housing and plunger rod, a compression spring 14, which is confined under tension between ring 11 and retaining piece 15 of cylindrical shape, is arranged around the plunger rod. The section 16 of this retaining piece has a diameter which is larger than the diameter of the bore 13 and a section 17 which, provided with turned screw thread, fits in the tapped screw thread section of bore 13.

The difference in diameter of the sections 16 and 17 of the retaining piece 15 produces a shoulder 18 as a result of which the retaining piece 15 can be screwed firmly against the pump housing, compression spring 14 at the same time being brought under a certain compression. This retaining piece is also provided with an axial bore having a diameter approximately equal to that of the plunger rod, as a result of which a guide is obtain ed for the plunger rod. A ring 19 made of flexible and resilient material and having an internal diameter approximately equal to that of the plunger rod and an external diameter equal to the external diameter of the section 16 of the retaining piece is fitted on the cylinder end of this retaining piece, which ring acts as a buffer for the plunger 3 against the retaining piece 15. A number of bores which have diameters which are not to be described in detail but are mutually identical and serve to maintain the atmospheric pressure between plunger and pump housing are fitted radially from the outside to the inside in the pump housing, at the height of section 16 of the retaining piece 15. Parallel to the plunger rod, the cylindrical pump housing is provided on its outside with a slot 20 in which a part of the pump lever 21 can retract. This pump lever is provided with a handle 22. Two or more fingers of one hand can be placed in the opening 23 thus produced. The pump lever 21 can partially hinge around a spindle 24 which sticks in axially and diametrically corresponding bores provided in the pump housing as well as in the centre of the circular part 26 of the pump lever 21. The slot 20 in line with the spindle 24 has a shape such that the circular part 26 of the pump lever fits therein and can partially rotate about spindle 24. The slot depth between positions 27 and 28 has a shape such that an open slotlike connection is produced with the bore for the plunger rod fitted in the pump housing. A slot which is delimited by the chord 30 and the associated part of the circumference of the circle is fitted in the circular segment 29 of the pump lever. The pump lever catch 31 which has a width approximately equal to the slot width is located in this slot. This pump lever catch 31 can partially hinge about a spindle 32 which is inserted into axially and diametrically corresponding bores in the circle segment 29 of the pump lever and the pump lever catch itself. A spring, not described in detail, between pump lever and pump lever catch ensures that the tooth of the pump lever catch is always under a light pressure in the direction of the plunger rod. The pump lever is formed such that a shoulder 33 which can activate the locking catch lever 34 is located thereon. This locking catch lever 34 is inserted as a good fit in the rearmost part of slot 20, has a buckled shape and can partially hinge about spindle 35 which is inserted into axially and diametrically corresponding bores provided in the pump housing as well as in the locking catch lever it self where this is buckled. The locking catch lever is provided at the pump lever end with a slot 36 in which a spring which fits around spindle 35 and which ensures that the locking catch lever, if not activated by shoulder 33 of the pump lever, occupies a position at all times which is determined by the buckled shape of the locking catch lever is fitted. On the other side of the spindle 35 the locking catch lever is connected with the locking catch 38.

The locking catch can be pushed as a good fit into a radial bore provided for this purpose in the pump housing. This bore 39 forms a connection between the outside of the pump housing and the bore for the plunger rod. The cylindrical locking catch 38 is provided at the plunger rod side with a face 40 perpendicular to the axial direction of the locking catch and covering half the circular surface of the locking catch. The shape of this face 40 is therefore delimited by the diameter and half the circumference of the locking catch. A second slanting face 41 is provided at the plunger rod side of the locking catch at an angle which is equal to the angle of the toothing 42 which is fitted over a certain length in the plunger rod. This slanting face 41 is delimited by half the ellipse which is produced by the slanting cylinder section of the locking catch and the diameter of the locking catch, the diameter delimitation of face 40 and the diameter delimitation of face 41 both lying in one and the same axial plane of the locking catch provided that the diameter delimitation of face 41 fits in the deepest point of the toothing of the plunger rod and that the distance from the diameter delimitation of face 40 is equal to the depth of the toothing of the plunger rod. A slot 43 in which the projecting part of the locking catch lever fits is provided in the locking catch at the height of locking catch lever. The depth of this slot is equal to half the diameter of the locking catch. The diametral delimitation of this slot is in the same plane as that of the diametral delimitations of the faces 40 and 41. This construction prevents the locking catch from being able to rotate in its bore. If the locking catch lever is moved by shoulder 33 of the pump lever in the direction of the plunger rod the other end of the locking catch will move in the opposite direction and take along the locking catch until the latter is pushed out of the toothing of the plunger rod. If the pump lever is rotated such that the shoulder 33 is released by the locking catch lever the latter will assume a position due to the compression of spring 37 such that the locking catch fits into the toothing of the plunger rod.

In order to prevent the plunger rod from being able to rotate in its bore the plunger rod is flattened at one side over a certain length. This flattening is located diametrically opposite the toothing 42 at the compression knob side of the plunger rod. A suitable locking plate which is located at the plunger rod side with the flat end approximately against the flattened part of the plunger rod is placed in a groove 44 which is provided radially in the pump housing and has the same width as the width of the flattening. This locking plate is fixed with two small bolts 65. At the other side of the pump housing opposite to slot 20 a second slot 45, in which the release lever 46 having a buckled shape fits, is present. This release lever can partially hinge about a spindle 47 which is inserted into axially and diametrically corresponding bores in the pump housing as well as the release lever where this is buckled. The release lever is connected at one end with the release catch 48. The construction, dimensions and mode of operation of this release catch are identical to catch 38 provided that the slanting face 41 of the locking catch is located at the plunger end of the locking catch and that the slanting face on the release catch is located at the compression knob side of the release catch. The release lever is provided with an annular handle. The thumb can be placed in the opening thus produced. An elongate recess 50 in which a spring 51 is placed by which the release lever, if it is not locked, assumes a certain position determined by the shape of the release lever is provided in the section 49 of the release lever at the plunger side end.

A ball 54 which fits in a cavity 70 which is matched to suit the ball shape and which is provided in this section 49 of the release lever is at the same time located at the plunger side of the release lever. This ball is kept under compression by a compression spring 55 and is pressed in the direction of the release lever. Ball 54 and compression spring 55 are located in a housing 56 which is provided with a bore 57. This bore narrows down at the edge of the housing 56 such that the ball can project only partially beyond the housing. The bore 57 is provided at the other side with a screw thread in which the retaining piece 58 fits. By rotating this retaining piece 58 the spring 55 is compressed and ball 54 is as a result put under pressure. Axially and diametrically corresponding bores 60 are fitted in the housing 56, the retaining piece 58 and the pump housing 1. The bore is provided in the pumphousing with screw thread and the housing 56 is connected with the pump housing by a suitable bolt 59. The housing 56 is partially retracted in the pump housing in a slot provided for this purpose on the dimensions of the housing. The release lever 46 can assume two positions. The position in which the section 49 comes to rest against the pump housing and in which the release catch 48 is pushed out of the pump housing as a result of the lever action and the position in which the section 52 comes to rest against the pump housing and by which the release catch is moved in the direction of the plunger rod. This position will not be assumed completely unless the plunger rod is pressed so far inwards by the compression knob 10 that the slanting face of the release catch is located opposite the slanting face of the single toothing 53 provided in the plunger rod, so that the release catch can shift into this toothing.

This position of the release lever remains for the time being secured by the compression of spring 51 and at the same time by the ball 54 which in this position of the release lever is pressed in a bowl-shaped cavity 61 of the lever. The two cavities 61 and 70 are identical in shape and dimension. A thin loose disposable diaphragm 25 having a diameter approximately equal to the diameter of the plunger 3 is located at the underside of the plunger. A bore 66 of very small diameter is located in the wall of the disposable cylinder 2 approximately at the bottom of the cylinder. This opening 66 is sealed air-tight by means of an easily removed adhesive tape. On removal of this adhesive tape an extremely narrow but open connection between the interior of the cylinder and the surrounding atmosphere is formed.

The instrument is prepared for use by rotating the pump lever 21 such that this is positioned at right angles on the pump housing as a result of which the shoulder 33 presses the locking catch lever 34 in and the locking catch 38 is pushed out of the toothing 42 of the plunger rod. At the same time, light pressure is exerted manually on the section 52 of the release lever 46 as a result of which the ball 54 is released from the bowl-shaped locking cavity 70 in which the ball was pressed. The release catch is pressed against the plunger rod by this manipulation. By pressing the compression knob 10 in the direction of the pump housing the single toothing 53 is at the same time moved in the direction of the release catch. As soon as this single toothing 53 has arrived at the same height as the release catch this will fit in the toothing and the compression knob can be released.

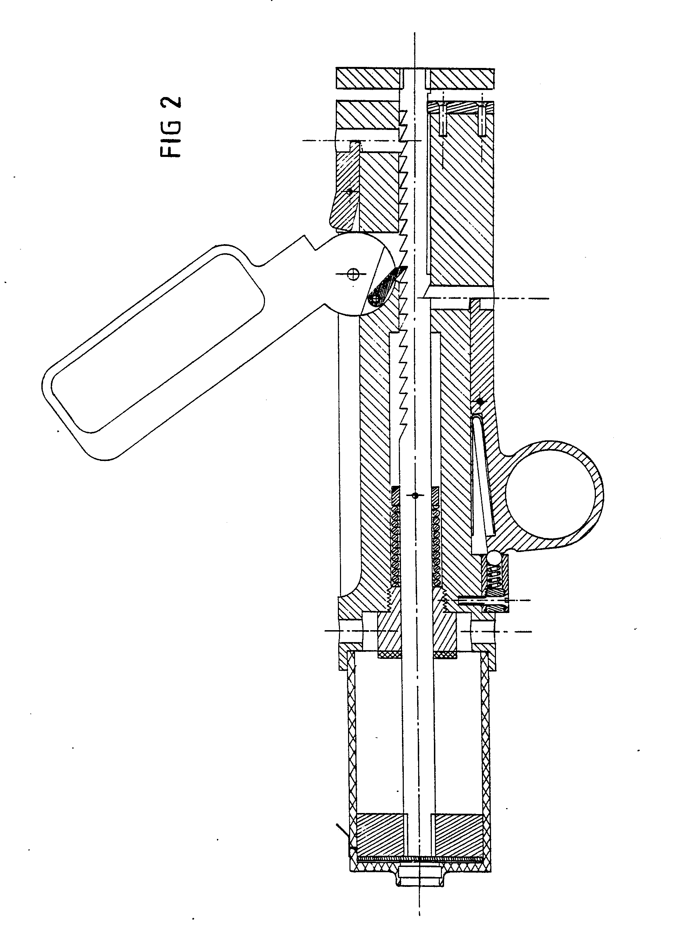

Figure 2 shows the instrument in longitudinal section and in a compressed state ready for operation. The pump lever 21 is now rotated such that the locking catch lever 34 is not activated and the locking catch 38 fits in the toothing 42 of the plunger rod.

The thumb is then placed in the opening 63 of the release lever and a few fingers of the same hand are placed in the opening 23 of the pump lever. The instrument is placed with the opening 5 centrically and perpendicularly to the most throbbing section of the abcess. The upright edge 6 at the same time serves for making contact with the skin all round. The release lever is then pressed in with the thumb as a result of which the release catch 48 is pushed out of the toothing 53. The tension of spring 14 on the ring 11 fastened on the plunger rod moves the plunger rod with the plunger connected thereto in the direction of the pump housing. The tension of spring 14 is such that the following frictions are overcome:

-Friction of plunger with the cylinder wall

-Friction of plunger rod with its bores in the pump housing, the retaining piece 15 and with the retaining plate 44.

-Friction of the toothing 42 with the pump lever catch 31 and the locking catch 38 which are both pressed under spring compression against the plunger rod.

At the same time, a vacuum is produced in the cylinder in the section between the plunger and the cylinder opening placed on the abcess. As a result of this, the skin of the abcess is drawn into the opening and comes into contact with the small knife placed diametrically therein. By this contact with the small knife the skin can be opened as a result of which the pus which emerges from the abcess can flow into the cylinder. If the abcess is not opened by the small knife because the vacuum in the cylinder is not high enough and/or the skin is too tough, the plunger can be moved with the aid of the pump lever further in the direction of the pump housing as a result of which a higher vacuum is produced in the cylinder which is sufficient for sucking the skin of the abcess with sufficient force against the small knife with the result that the skin is incised and the pus can flow out. As long as the pus is drained out of the abcess by the light vacuum caused by the compression of spring 14 on the plunger the plunger moves in the direction of the pump housing. As soon as this movement stops, however, because the light vacuum is no longer sufficient for draining the pus out of the abcess the plunger is moved by means of the pump lever further in the direction of the pump housing as a result of which a high to very high vacuum is produced in the cylinder which ensures that all the pus present is removed from the abcess. During this pumping action, the plunger can move only in the direction of the pump housing because the locking catch fits in the next toothing of the plunger rod after each pump movement and the plunger can therefore not move back in the direction of the abcess. After all the pus present has been drained from the abcess the adhesive tape on the small opening in the cylinder wall is removed as a result of which the vacuum in the cylinder is gradually reduced to the pressure of the surrounding atmosphere, the sucking action being slowly cancelled and the instrument can be removed from the wound. At the instant at which the opening 5 is released from the skin the plunger will move again under the compression of spring 14 in the direction of the pump housing as a result of which the already present pus in the cylinder is sucked further into the cylinder until the upper end of the plunger comes to a standstill against the stop 19. Immediately after breaking of the contact with the skin the instrument should be held vertically with the cylinder opening 5 directed upwards; this is to prevent pus from flowing out via this opening. The instrument is turned above a suitable place for the purpose so that the opening of the cylinder is directed downwards.

The cylinder is removed from the pump housing and is discarded together with its content of pus and the loose diaphragm. A sterile cylinder with associated diaphragm is pushed over the plunger until this is clamped in the appropriate cylindrical recess 4 of the pump housing.

The instrument is now again ready for use.

It is, moreover, possible to fit both the cylinder 2 and plunger 3 so as to be quickly exchangeable on the device. After use, in this case not only cylinder 2 is discarded but plunger 3 (with the diaphragm 25 which may be present) is also discarded.

The fixing of plunger 3 and cylinder 2 can be realised in any manner known in the state of the art for quick-acting closures, such as bayonet couplings.

Although a preferred embodiment of the invention has been described above it is to be understood that any type of variation may be provided which, for those skilled in the state of the art, falls within the present invention.

## Claims

1. Device for incising and draining an abcess, comprising a holder which is at least partially open at one end and which is to be placed around the abcess, this holder being provided at the open side with cutting means which in the fitted state are located at a distance from the abcess and means being present for providing a vacuum in this holder.

2. Device according to Claim 1, in which the means which provide a vacuum comprise a plunger/cylinder assembly.

3. Device according to Claim 2, in which the plunger/cylinder assembly forms part of the holder.

4. Device according to Claim 3, in which the plunger is provided with a diaphragm which is fitted together with the cylinder simply detachably on the device.

5. Device according to Claim 3 or 4, in which the plunger is fitted on the device so as to be simply detachable.

6. Device according to one of the preceding claims, in which the means for providing a vacuum are equipped such that during use of the device a first vacuum is applied during a first phase and a second more considerable vacuum is applied during a second subsequent phase.

7. Device according to Claim 6 at least in combination with Claim 2, in which the first vacuum is generated by spring means acting on the plunger/cylinder assembly the second vacuum is generated by lever means.

8. Device according to one of the preceding claims, in which the device, with the exception of the quickly detachable cylinder and diaphragm which may be present, consists of sterilisable material.

9. Device according to one of the preceding claims, comprising a pump housing (1) and, fitted therein, a plunger rod (9) provided at one end with a plunger (3) and at the other end provided with a compression knob (10), the pump housing (1) being provided at the side next to the plunger (3) with means for fixing a cylinder (2), spring means (20) engaging on the plunger rod being present in the pump housing (1) and the plunger rod (9) being provided with a toothing (42) to be engaged with a transport mechanism.

10. Device according to Claim 9, in which a locking mechanism is present for locking the plunger rod, under compression of the spring means (14).

11. Device according to Claim 9 or 10 in which the transport mechanism comprises a lever mechanism engaging the toothing in one direction and means which hinder the movement in the opposite direction.

FIG 1

5.  7. 66.  25.  4. 16 15. 18. 17.  9. 20 14.  13. 12. 11. 32 29. 30. 24. 33.  21.  22.  23.  34.  43.

38.

41.
40.
39.

31.  35.

42.

27.  28.

63.

8. 6.  2.  3. 19. 58. 59. 60. 56. 55. 57. 54. 70. 61. 62. 49. 50. 51. 46. 47. 52. 45.  48.  1. 26.  36. 37.  53. 44.  65. 64.  10.

0 287 186

FIG 2

0 287 186